(19) Europäisches Patentamt
European Patent Office
Office européen des brevets

(11) Veröffentlichungsnummer: **0 560 162 A1**

# (12) EUROPÄISCHE PATENTANMELDUNG

(21) Anmeldenummer: **93103217.1**

(22) Anmeldetag: **01.03.93**

(51) Int. Cl.$^5$: **C07D 233/68**, A61K 31/415, //C07D233/66,C07D233/92

(30) Priorität: **13.03.92 DE 4208052**

(43) Veröffentlichungstag der Anmeldung: **15.09.93 Patentblatt 93/37**

(84) Benannte Vertragsstaaten: **AT BE CH DE DK ES FR GB GR IE IT LI LU MC NL PT SE**

(71) Anmelder: **BAYER AG**

**D-51368 Leverkusen(DE)**

(72) Erfinder: **Müller, Ulrich, Dr.**
**Claudiusweg 9**
**W-5600 Wuppertal(DE)**
Erfinder: **Müller-Gliemann, Matthias, Dr.**
**Laibacher Strasse 10**
**W-5650 Solingen-Ohligs(DE)**
Erfinder: **Dressel, Jürgen, Ph. D.**
**Claudiusweg 9**
**W-5600 Wuppertal(DE)**
Erfinder: **Fey, Peter, Dr.**
**Am Eickhof 23**
**W-5600 Wuppertal(DE)**
Erfinder: **Hanko, Rudolf, Dr.**
**Schillerstrasse 23**
**W-4000 Düsseldorf(DE)**
Erfinder: **Hübsch, Walter, Dr.**
**Wildsteig 22**
**W-5600 Wuppertal(DE)**
Erfinder: **Krämer, Thomas, Dr.**
**In den Birken 92a**
**W-5600 Wuppertal(DE)**
Erfinder: **Niewöhner, Ulrich, Dr.**
**Gartenstrasse 3**
**W-5632 Wermelskirchen(DE)**
Erfinder: **Beuck, Martin, Dr.**
**Trills 7**
**W-4006 Erkrath 2(DE)**
Erfinder: **Kazda, Stanislav, Prof. Dr.**
**Gellertweg 18**
**W-5600 Wuppertal(DE)**
Erfinder: **Wohlfeil, Stefan, Dr.**
**Tucherweg 25**
**W-4010 Hilden(DE)**
Erfinder: **Yalkinoglu, Özkan, Dr.**
**Neuer Weg 21**
**W-5600 Wuppertal(DE)**
Erfinder: **Knorr, Andreas, Dr.**
**Trillser Graben 10**
**W-4006 Erkrath 2(DE)**
Erfinder: **Stasch, Johannes-Peter, Dr.**
**Schneewittchenweg 37**
**W-5600 Wuppertal(DE)**

(54) **Imidazolylmethyl-substituierte Phenylessigsäureamide, Verfahren zu ihrer Herstellung und ihre Verwendung in Arzneimitteln.**

(57) Imidazolyl-substituierte Phenylessigsäureamide der allgemeinen Formel

EP 0 560 162 A1

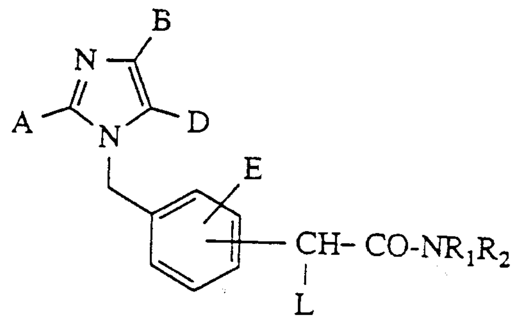

(I),

in welcher

A für geradkettiges oder verzweigtes Alkyl oder Alkenyl mit jeweils bis zu 8 Kohlenstoffatomen steht, oder für Cycloalkyl mit 3 bis 8 Kohlenstoffatomen steht,

B für Wasserstoff, Halogen oder für Perfluoralkyl mit bis zu 5 Kohlenstoffatomen steht,

D für eine Gruppe der Formel $-CH_2OR^3$, $-CO-R^4$, $-CO-NR^5R^6$,

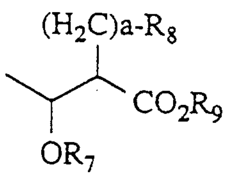

oder

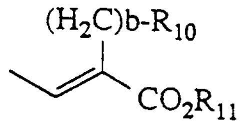

steht,

E für Wasserstoff, Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano oder Carboxy steht,

L für geradkettiges oder verzweigtes Alkyl mit bis zu 8 Kohlenstoffatomen steht, das gegebenenfalls durch Cycloalkyl mit 3 bis 8 Kohlenstoffatomen oder Phenyl substituiert ist,
für Cycloalkyl mit 3 bis 12 Kohlenstoffatomen steht, das gegebenenfalls durch geradkettiges oder verzweigtes Alkyl mit bis zu 6 Kohlenstoffatomen substituiert ist,

$R^1$ für Wasserstoff oder für geradkettiges oder verzweigtes Alkyl mit bis zu 8 Kohlenstoffatomen steht,

$R^2$ für einen Rest der Formel $-C(CH_3)_2-CH_2OH$,

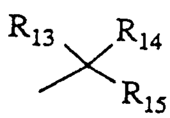

,

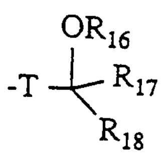

,

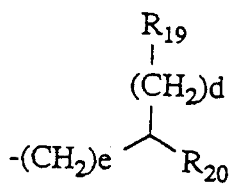

,

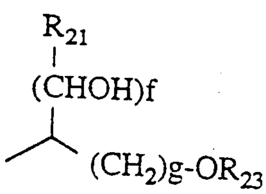

oder

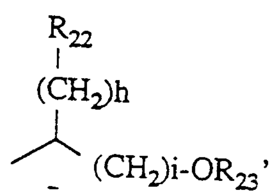

steht, und deren Salze.

Die Verbindungen besitzen Angiotensin II - antagonistische Wirkung und können als Wirkstoffe in Arzneimitteln, insbesondere zur Behandlung von Hypertonie und Atherosklerose eingesetzt werden.

Die Erfindung betrifft Imidazolyl-substituierte Phenylessigsäureamide, Verfahren zu ihrer Herstellung sowie ihre Verwendung in Arzneimitteln, insbesondere als blutdrucksenkende und antiatherosklerotische Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksteigernden Oktapeptid Angiotensin II abgebaut wird. Die verschiedenen Effekte des Angiotensin II, wie beispielsweise Vasokonstriktion, $Na^+$-Retention in der Niere, Aldosteronfreisetzung in der Nebenniere und Tonuserhöhung des sympathischen Nervensystems wirken synergistisch im Sinne einer Blutdruckerhöhung.

Darüber hinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Außerdem sind aus den Publikationen EP 407 102, EP 399 731; EP 399 732 und EP 324 347 heterocyclische Verbindungen mit A II-antagonistischer Wirkung bekannt.

Die Erfindung betrifft Imidazolyl-substituierte Phenylessigsäureamide der allgemeinen Formel (I)

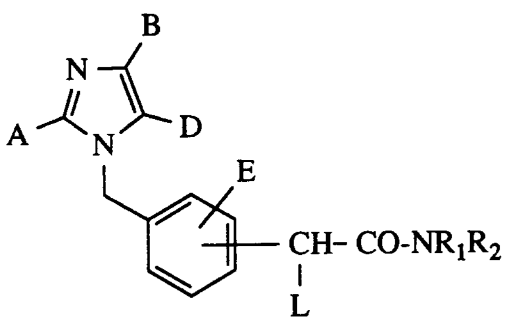

(I),

in welcher

A für geradkettiges oder verzweigtes Alkyl oder Alkenyl mit jeweils bis zu 8 Kohlenstoffatomen steht, oder
für Cycloalkyl mit 3 bis 8 Kohlenstoffatomen steht,

B für Wasserstoff, Halogen oder für Perfluoralkyl mit bis zu 5 Kohlenstoffatomen steht,

D für eine Gruppe der Formel $-CH_2OR^3$, $-CO-R^4$, $-CO-NR^5R^6$,

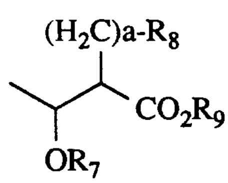

oder

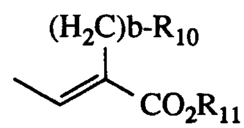

steht,
worin

$R^3$ Wasserstoff oder geradkettiges oder verzweigtes Alkyl mit bis zu 8 Kohlenstoffatomen bedeutet,

$R^4$ Wasserstoff, Hydroxy oder geradkettiges oder verzweigtes Alkoxy mit bis zu 8 Kohlenstoffatomen bedeutet,

$R^5$ und $R^6$ gleich oder verschieden sind und Wasserstoff, geradkettiges oder verzweigtes Alkyl mit bis zu 8 Kohlenstoffatomen bedeuten,
oder

$R^5$ die oben angegebene Bedeutung hat
und

$R^6$ eine Gruppe der Formel $-SO_2R^{12}$ bedeutet,
worin

$R^{12}$ geradkettiges oder verzweigtes Alkyl mit bis zu 6 Kohlenstoffatomen, Benzyl oder Phenyl bedeutet, die gegebenenfalls durch geradkettiges oder verzweigtes Alkyl mit bis zu 6 Kohlenstoffatomen substituiert sind,

a und b gleich oder verschieden sind und eine Zahl 0, 1 oder 2 bedeuten,

$R^7$ Wasserstoff oder geradkettiges oder verzweigtes Alkyl mit bis zu 8 Kohlenstoffatomen oder eine Hydroxyschutzgruppe bedeutet,

$R^8$ und $R^{10}$ gleich oder verschieden sind und Wasserstoff, geradkettiges oder verzweigtes Alkyl mit bis zu 6 Kohlenstoffatomen, Cycloalkyl mit 3 bis 8 Kohlenstoffatomen, Phenyl oder Thienyl bedeuten,

$R^9$ und $R^{11}$ gleich oder verschieden sind und Wasserstoff oder geradkettiges oder verzweigtes Alkyl mit bis zu 8 Kohlenstoffatomen bedeuten,

E für Wasserstoff, Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano oder Carboxy steht,

L für geradkettiges oder verzweigtes Alkyl mit bis zu 8 Kohlenstoffatomen steht, das gegebenenfalls durch Cycloalkyl mit 3 bis 8 Kohlenstoffatomen oder Phenyl substituiert ist,

für Cycloalkyl mit 3 bis 12 Kohlenstoffatomen steht, das gegebenenfalls durch geradkettiges oder verzweigtes Alkyl mit bis zu 6 Kohlenstoffatomen substituiert ist,

$R^1$ für Wasserstoff oder für geradkettiges oder verzweigtes Alkyl mit bis zu 8 Kohlenstoffatomen steht,

$R^2$ für einen Rest der Formel $-C(CH_3)_2-CH_2OH$,

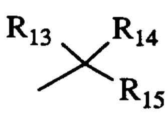 , 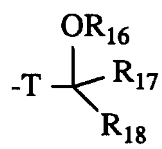 , 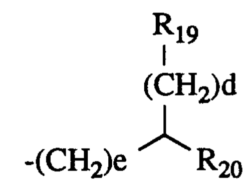 ,

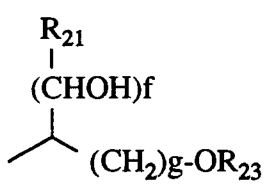 oder 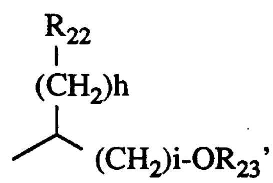

steht,
worin

$R^{13}$ und $R^{14}$ gleich oder verschieden sind und Wasserstoff oder geradkettiges oder verzweigtes Alkyl mit bis zu 8 Kohlenstoffatomen bedeuten,

$R^{15}$ geradkettiges oder verzweigtes Alkyl mit 2 bis 8 Kohlenstoffatomen bedeutet, das, gegebenenfalls auch bis zu 3-fach gleich oder verschieden durch Hydroxy, Carboxy, Trifluormethyl, Halogen, Nitro, Cyano, durch geradkettiges oder verzweigtes Alkoxy, Acyl oder Alkoxycarbonyl mit jeweils bis zu 8 Kohlenstoffatomen oder durch Aryl mit 6 bis 10 Kohlenstoffatomen oder einen 5-bis 7-gliedrigen, gesättigten oder ungesättigten Heterocyclus oder benzokondensierten Heterocyclus mit bis zu 3 Heteroatomen aus der Reihe S, N oder O substituiert sein muß, welche ihrerseits bis zu 2-fach gleich oder verschieden Halogen, Nitro, Cyano, Hydroxy oder durch geradkettiges oder verzweigtes Alkyl oder Alkoxy mit jeweils bis zu 6 Kohlenstoffatomen substituiert sein können, oder

Alkyl gegebenenfalls durch eine Gruppe der Formel $-CO-NR^{24}R^{25}$ substituiert ist,
worin

$R^{24}$ und $R^{25}$ die oben angegebene Bedeutung von $R^5$ und $R^6$ haben und mit dieser gleich oder verschieden sind, oder

| | |
|---|---|
| $R^{24}$ und $R^{25}$ | gemeinsam mit dem Stickstoffatom einen 5- bis 7-gliedrigen, gesättigten oder ungesättigten Heterocyclus mit bis zu 2 weiteren Heteroatomen aus der Reihe S, N oder O bilden, |
| | oder |
| $R^{15}$ | geradkettiges oder verzweigtes Alkoxy oder Alkoxycarbonyl mit jeweils bis zu 8 Kohlenstoffatomen, Hydroxy, Carboxy, Trifluormethyl oder die Gruppe der Formel $-CO-NR^{24}R^{25}$ bedeutet, |
| | worin |
| $R^{24}$ und $R^{25}$ | die oben angegebene Bedeutung haben, |
| T | geradkettiges oder verzweigtes Alkyl mit 2 bis zu 8 Kohlenstoffatomen bedeutet, |
| $R^{16}$ | Wasserstoff oder geradkettiges oder verzweigtes Alkyl mit bis zu 8 Kohlenstoffatomen bedeutet, |
| $R^{17}$ und $R^{18}$ | gleich oder verschieden sind und Wasserstoff, geradkettiges oder verzweigtes Alkyl mit bis zu 6 Kohlenstoffatomen oder Phenyl bedeuten, |
| | oder |
| $R^{17}$ und $R^{18}$ | gemeinsam einen gesättigten und/oder ungesättigten carbocyclischen Bi- oder Tricyclus bilden, |
| d | eine Zahl 0, 1, 2, 3 oder 4 bedeutet, |
| e | eine Zahl 1, 2, 3 oder 4 bedeutet, |
| f | eine Zahl 1, 2, 3 oder 4 bedeutet, |
| g | eine Zahl 1, 2, 3, 4, 5 oder 6 bedeutet, |
| h | eine Zahl 0, 1, 2, 3 oder 4 bedeutet, |
| i | eine Zahl 2, 3, 4 oder 5 bedeutet, |
| $R^{20}$ | die oben angegebene Bedeutung von $R^{15}$ hat und mit dieser gleich oder verschieden ist oder die $-CH_2OH$-Gruppe bedeutet, |
| $R^{19}$, $R^{21}$ und $R^{22}$ | gleich oder verschieden sind und Phenyl bedeuten, das gegebenenfalls bis zu 3-fach gleich oder verschieden durch Halogen, Hydroxy, Trifluormethyl oder durch geradkettiges oder verzweigtes Alkyl oder Alkoxy mit jeweils bis zu 8 Kohlenstoffatomen, Phenoxy oder Benzyloxy substituiert ist, |
| $R^{23}$ und $R^{23'}$ | gleich oder verschieden sind und Wasserstoff, geradkettiges oder verzweigtes Alkyl oder Alkoxycarbonyl mit jeweils bis zu 8 Kohlenstoffatomen bedeuten, |

und deren Salze.

Die erfindungsgemäßen Verbindungen der allgemeinen Formel (I) können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der heterocyclisch substituierten Phenylessigsäurederivate können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium-oder Calciumsalze, sowie Ammoniumsalze, die abgeleitet sind von Ammoniak, oder organischen Aminen wie beispielsweise Ethylamin, Di- bzw. Triethylamin, Di-bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweiligen Mischungen. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Heterocyclus oder benzokondensierter Heterocyclus steht im allgemeinen für einen 5- bis 7-gliedrigen, bevorzugt 5- bis 6-gliedrigen, gesättigten oder ungesättigten Ring, der als Heteroatome bis zu 2 Sauerstoff-, Schwefel- und/oder Stickstoffatome enthalten kann. Bevorzugt sind 5- und 6-gliedrige Ringe mit einem Sauerstoff-, Schwefel und/oder bis zu 2 Stickstoffatomen. Bevorzugt werden genannt: Thienyl, Furyl, Pyrrolyl, Pyrazolyl, Pyridyl, Chinolinyl, Tetrahydrofuranyl, Tetrahydropyranyl, Dihydrobenzopyranyl oder Dihydrobenzofuranyl. Bevorzugt sind Pyridyl, Furanyl, Thienyl, Tetrahydrofuranyl oder Pyrrolidinyl.

Carbocyclischer Bi- und Tricyclus steht im allgemeinen für Fluorenyl, Naphthyl, Indenyl, Anthranyl oder Phenanthryl. Bevorzugt sind Indenyl und Fluorenyl.

Hydroxyschutzgruppe im Rahmen der oben angegebenen Definition steht im allgemeinen für eine Schutzgruppe aus der Reihe: Trimethylsilyl, Triethylsilyl, Triisopropylsilyl, tert.Butyl-dimethylsilyl, tert.-Butyldiphenylsilyl, Trimethylsilylethoxycarbonyl,Benzyl, Triphenylmethyl(Trityl), Monomethoxytrityl (MMTr), Dimethoxytrityl (DMTr),Benzyloxycarbonyl, 2-Nitrobenzyl, 4-Nitrobenzyl, 2-Nitrobenzyloxycarbonyl, 4-Nitrobenzyloxycarbonyl, tert. Butyloxycarbonyl, 4-Methoxybenzyl, 4-Methoxybenzyloxycarbonyl, Formyl, Acetyl, Trichloracetyl, 2,2,2-Trichlorethoxycarbonyl, 2,4-Dimethoxybenzyl, 2,4-Dimethoxybenzyloxycarbonyl, Methoxymethyl, Methylthiomethyl, Methoxyethoxymethyl, [2-(Trimethylsilyl)ethoxy]methyl, 2-(Methylthiomethoxy)ethoxycarbonyl, Tetrahydropyranyl, Benzoyl, 4-Methylbenzoyl, 4-Nitrobenzoyl, 4-Fluorbenzoyl, 4-Chlorbenzoyl oder 4-Methoxybenzoyl. Bevorzugt ist Acetyl.

Bevorzugt sind Verbindungen der allgemeinen Formel (I),
in welcher

A für geradkettiges oder verzweigtes Alkyl oder Alkenyl mit jeweils bis zu 6 Kohlenstoffatomen steht, oder
für Cyclopropyl, Cyclobutyl, Cyclopentyl, Cyclohexyl oder Cycloheptyl steht,

B für Wasserstoff, Fluor, Chlor, Brom oder für Perfluoralkyl mit bis zu 4 Kohlenstoffatomen steht,

D für eine Gruppe der Formel $-CH_2OR^3$, $-CO-R^4$, $-CO-NR^5R^6$,

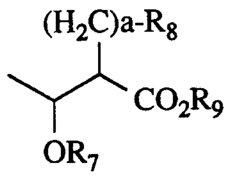

oder

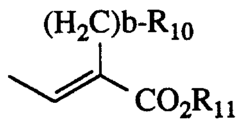

steht,
worin

$R^3$ Wasserstoff oder geradkettiges oder verzweigtes Alkyl mit bis zu 6 Kohlenstoffatomen bedeutet,

$R^4$ Wasserstoff, Hydroxy oder geradkettiges oder verzweigtes Alkoxy mit bis zu 6 Kohlenstoffatomen bedeutet,

$R^5$ und $R^6$ gleich oder verschieden sind und Wasserstoff, geradkettiges oder verzweigtes Alkyl mit bis zu 6 Kohlenstoffatomen bedeuten,
oder

$R^5$ die oben angegebene Bedeutung hat
und

$R^6$ eine Gruppe der Formel $-SO_2R^{12}$ bedeutet,
worin

$R^{12}$ geradkettiges oder verzweigtes Alkyl mit bis zu 4 Kohlenstoffatomen, Benzyl oder Phenyl bedeutet, die gegebenenfalls durch geradkettiges oder verzweigtes Alkyl mit bis zu 4 Kohlenstoffatomen substituiert sind,

a und b gleich oder verschieden sind und eine Zahl 0 oder 1 bedeuten,

$R^7$ Wasserstoff oder geradkettiges oder verzweigtes Alkyl mit bis zu 6 Kohlenstoffatomen oder Acetyl bedeutet,

$R^8$ und $R^{10}$ gleich oder verschieden sind und geradkettiges oder verzweigtes Alkyl mit bis zu 4 Kohlenstoffatomen, Cyclopropyl, Cyclobutyl, Cyclopentyl, Cyclohexyl oder Cycloheptyl, Phenyl oder Thienyl bedeuten,

$R^9$ und $R^{11}$ gleich oder verschieden sind und Wasserstoff oder geradkettiges oder verzweigtes Alkyl mit bis zu 6 Kohlenstoffatomen bedeuten,

E für Wasserstoff, Fluor, Chlor, Brom, Trifluormethyl, Carboxy oder geradkettiges oder verzweigtes Alkyl, Alkoxy oder Alkoxycarbonyl mit jeweils bis zu 4 Kohlenstoffatomen steht,

L für geradkettiges oder verzweigtes Alkyl mit bis zu 6 Kohlenstoffatomen steht, das gegebenenfalls durch Cyclopropyl, Cyclobutyl, Cyclopentyl, Cyclohexyl, Cycloheptyl, Cyclooctyl oder Phenyl substituiert ist,

für Cyclopropyl, Cyclobutyl, Cyclopentyl, Cyclohexyl, Cycloheptyl oder Cyclooctyl steht, die gegebenenfalls durch geradkettiges oder verzweigtes Alkyl mit bis zu 4 Kohlenstoffatomen substituiert sind,

$R^1$ für Wasserstoff oder für geradkettiges oder verzweigtes Alkyl mit bis zu 6 Kohlenstoffatomen steht,

$R^2$ für einen Rest der Formel $-C(CH_3)_2-CH_2OH$,

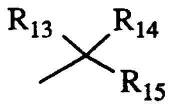 , 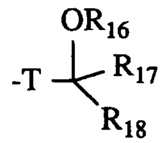 , 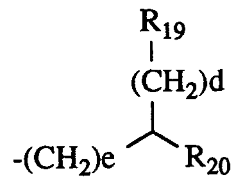 ,

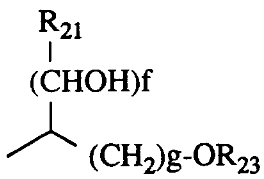 oder 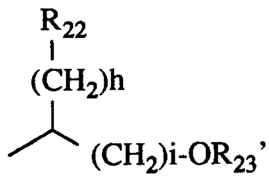

steht,

worin

$R^{13}$ und $R^{14}$ gleich oder verschieden sind und Wasserstoff oder geradkettiges oder verzweigtes Alkyl mit bis zu 6 Kohlenstoffatomen bedeuten,

$R^{15}$ geradkettiges oder verzweigtes Alkyl mit 2 bis 6 Kohlenstoffatomen bedeutet, das gegebenenfalls auch bis zu 2-fach gleich oder verschieden, durch Hydroxy, Carboxy, Trifluormethyl, Fluor, Chlor, Brom, durch geradkettiges oder verzweigtes Alkoxy, Acyl oder Alkoxycarbonyl mit jeweils bis zu 6 Kohlenstoffatomen oder durch Phenyl, Naphthyl, Furanyl, Pyrrolidinyl, Thienyl, Pyridyl, Tetrahydrofuranyl, Tetrahydropyranyl, Dihydrobenzopyranyl oder Dihydrobenzofuranyl substituiert sein muß, die gegebenenfalls durch Fluor, Chlor, Hydroxy substituiert sein können, oder

Alkyl gegebenenfalls durch eine Gruppe der Formel $-CO-NR^{24}R^{25}$ substituiert sein kann,

worin

$R^{24}$ und $R^{25}$ die oben angegebene Bedeutung von $R^5$ und $R^6$ haben und mit dieser gleich oder verschieden sind, oder

$R^{24}$ und $R^{25}$ gemeinsam mit dem Stickstoffatom einen Morpholinring bilden,

oder

$R^{15}$ geradkettiges oder verzweigtes Alkoxy oder Alkoxycarbonyl mit jeweils bis zu 6 Kohlenstoffatomen, Hydroxy, Carboxy, Trifluormethyl oder die Gruppe der Formel $-CO-NR^{24}R^{25}$ bedeutet,

worin

$R^{24}$ und $R^{25}$ die oben angegebene Bedeutung haben,

T geradkettiges oder verzweigtes Alkyl mit 2 bis zu 6 Kohlenstoffatomen bedeutet,

$R^{16}$ Wasserstoff oder geradkettiges oder verzweigtes Alkyl mit bis zu 6 Kohlenstoffatomen bedeutet,

$R^{17}$ und $R^{18}$ gleich oder verschieden sind Wasserstoff, geradkettiges oder verzweigtes Alkyl mit bis zu 4 Kohlenstoffatomen oder Phenyl bedeuten,

oder

$R^{17}$ und $R^{18}$ gemeinsam einen Indenyl- oder Fluorenylring bilden,

d eine Zahl 0, 1, 2 oder 3 bedeutet,

e eine Zahl 1, 2 oder 3 bedeutet,

f eine Zahl 1, 2 oder 3 bedeutet,

g eine Zahl 1, 2, 3, 4 oder 5 bedeutet,

h eine Zahl 0, 1, 2 oder 3 bedeutet,

i eine Zahl 2, 3 oder 4 bedeutet,

| | |
|---|---|
| $R^{20}$ | die oben angegebene Bedeutung von $R^{15}$ hat und mit dieser gleich oder verschieden ist oder die $-CH_2OH$-Gruppe bedeutet, |
| $R^{19}$, $R^{21}$ und $R^{22}$ | gleich oder verschieden sind und Phenyl bedeuten, das gegebenenfalls bis zu 2-fach gleich oder verschieden durch Fluor, Chlor, Brom, Hydroxy, Trifluormethyl oder durch geradkettiges oder verzweigtes Alkyl oder Alkoxy mit jeweils bis zu 6 Kohlenstoffatomen, Phenoxy oder Benzyloxy substituiert ist, |
| $R^{23}$ und $R^{23'}$ | gleich oder verschieden sind und Wasserstoff oder geradkettiges oder verzweigtes Alkyl oder Alkoxycarbonyl mit jeweils bis zu 6 Kohlenstoffatomen bedeutet, |

und deren Salze.

Besonders bevorzugt sind Verbindungen der allgemeinen Formel (I), in welcher

| | |
|---|---|
| A | für geradkettiges oder verzweigtes Alkyl oder Alkenyl mit jeweils bis zu 4 Kohlenstoffatomen steht, oder<br>für Cyclopropyl, Cyclopentyl oder Cyclohexyl steht, |
| B | für Wasserstoff, Fluor, Chlor oder für Perfluoralkyl mit bis zu 2 Kohlenstoffatomen steht, |
| D | für eine Gruppe der Formel $-CH_2OR^3$, $-CO-R^4$, $-CO-NR^5R^6$, |

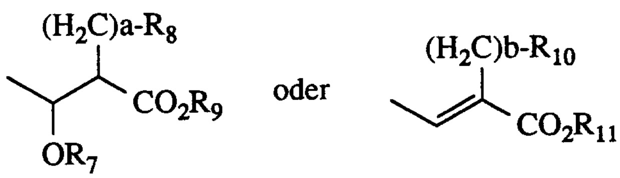

| | |
|---|---|
| | steht,<br>worin |
| $R^3$ | Wasserstoff oder geradkettiges oder verzweigtes Alkyl mit bis zu 4 Kohlenstoffatomen bedeutet, |
| $R^4$ | Wasserstoff, Hydroxy oder geradkettiges oder verzweigtes Alkoxy mit bis zu 4 Kohlenstoffatomen bedeutet, |
| $R^5$ und $R^6$ | gleich oder verschieden sind und Wasserstoff, geradkettiges oder verzweigtes Alkyl mit bis zu 4 Kohlenstoffatomen bedeuten,<br>oder |
| $R^5$ | die oben angegebene Bedeutung hat<br>und |
| $R^6$ | eine Gruppe der Formel $-SO_2R^{12}$ bedeutet,<br>worin |
| $R^{12}$ | Methyl, Ethyl, Benzyl, p-Tolyl oder Phenyl bedeutet, |
| a und b | gleich oder verschieden sind und eine Zahl 0 oder 1 bedeuten, |
| $R^7$ | Wasserstoff oder geradkettiges oder verzweigtes Alkyl mit bis zu 4 Kohlenstoffatomen bedeutet, |
| $R^8$ und $R^{10}$ | gleich oder verschieden sind und Cyclopropyl, Cyclohexyl oder Phenyl bedeuten, |
| $R^9$ und $R^{11}$ | gleich oder verschieden sind und Wasserstoff oder geradkettiges oder verzweigtes Alkyl mit bis zu 4 Kohlenstoffatomen bedeuten, |
| E | für Wasserstoff, Fluor, Chlor, Brom, Trifluormethyl, Trifluormethoxy oder Methyl steht, |
| L | für geradkettiges oder verzweigtes Alkyl mit bis zu 4 Kohlenstoffatomen steht, das gegebenenfalls durch Cyclopropyl, Cyclopentyl, Cyclohexyl, Cycloheptyl oder Phenyl substituiert ist,<br>für Cyclopropyl, Cyclopentyl, Cyclohexyl,Cycloheptyl oder Cyclooctyl steht, |
| $R^1$ | für Wasserstoff oder für geradkettiges oder verzweigtes Alkyl mit bis zu 4 Kohlenstoffatomen steht, |
| $R^2$ | für einen Rest der Formel $-C(CH_3)_2-CH_2OH$ |

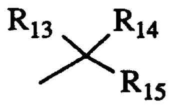 , 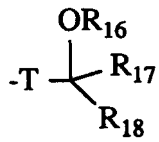 , 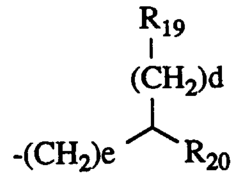 ,

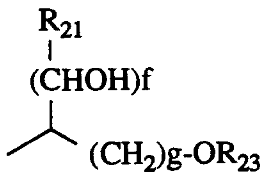 oder 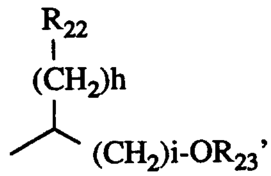

steht,
worin

| | |
|---|---|
| $R^{13}$ und $R^{14}$ | gleich oder verschieden sind und Wasserstoff oder geradkettiges oder verzweigtes Alkyl mit bis zu 4 Kohlenstoffatomen bedeuten, |
| $R^{15}$ | geradkettiges oder verzweigtes Alkyl mit 2 bis 4 Kohlenstoffatomen bedeutet, das durch Hydroxy, Carboxy, Trifluormethyl, geradkettiges oder verzweigtes Alkoxy, Acyl oder Alkoxycarbonyl mit jeweils bis zu 4 Kohlenstoffatomen oder durch Phenyl, Pyridyl, Furanyl, Thienyl, Tetrahydrofuranyl oder Pyrrolidinyl substituiert sein muß, oder |
| | geradkettiges oder verzweigtes Alkoxy oder Alkoxycarbonyl mit jeweils bis zu 4 Kohlenstoffatomen, Hydroxy, Carboxy oder Trifluormethyl bedeutet, |
| T | geradkettiges oder verzweigtes Alkyl mit 2 bis zu 5 Kohlenstoffatomen bedeutet, |
| $R^{16}$ | Wasserstoff oder geradkettiges oder verzweigtes Alkyl mit bis zu 4 Kohlenstoffatomen bedeutet, |
| $R^{17}$ und $R^{18}$ | gleich oder verschieden sind und Wasserstoff, Methyl, Ethyl oder Phenyl bedeuten, oder |
| $R^{17}$ und $R^{18}$ | gemeinsam einen Indenyl- oder Fluorenylring bilden, |
| d | eine Zahl 0, 1 oder 2 bedeutet, |
| e | eine Zahl 1, 2 oder 3 bedeutet, |
| f | eine Zahl 1, 2 oder 3 bedeutet, |
| g | eine Zahl 1, 2, 3 oder 4 bedeutet, |
| h | eine Zahl 0, 1 oder 2 bedeutet, |
| i | eine Zahl 2 oder 3 bedeutet, |
| $R^{20}$ | die oben angegebene Bedeutung von $R^{15}$ hat und mit dieser gleich oder verschieden ist oder die $-CH_2OH$-Gruppe bedeutet, |
| $R^{19}$, $R^{21}$ und $R^{22}$ | gleich oder verschieden sind und Phenyl bedeuten, das gegebenenfalls durch Fluor, Hydroxy, Trifluormethyl oder durch geradkettiges oder verzweigtes Alkyl oder Alkoxy mit jeweils bis zu 4 Kohlenstoffatomen substituiert ist, |
| $R^{23}$ und $R^{23'}$ | gleich oder verschieden sind und Wasserstoff oder geradkettiges oder verzweigtes Alkyl oder Alkoxycarbonyl mit jeweils bis zu 4 Kohlenstoffatomen bedeuten, |

und deren Salze.

Außerdem wurde ein Verfahren zur Herstellung der erfindungsgemäßen Verbindungen der allgemeinen Formel (I) gefunden, das dadurch gekennzeichnet ist, daß man

Verbindungen der allgemeinen Formel (II)

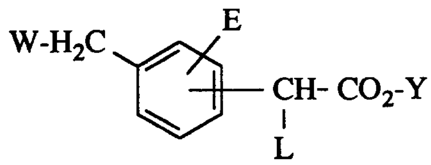

(II),

in welcher

E und L die oben angegebene Bedeutung haben

W für eine typische Abgangsgruppe wie beispielsweise Chlor, Brom, Jod, Tosylat oder Mesylat, vorzugsweise für Brom steht

und

Y für $C_1$-$C_6$-Alkyl steht,

zunächst mit Imidazolen der allgemeinen Formel (III)

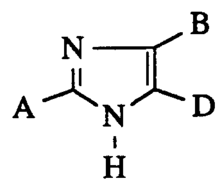

(III),

in welcher

A, B und D die oben angegebene Bedeutung haben,

in inerten Lösemitteln, gegebenenfalls in Anwesenheit einer Base und gegebenenfalls unter Schutzgasatmosphäre zu Verbindungen der allgemeinen Formel (IV)

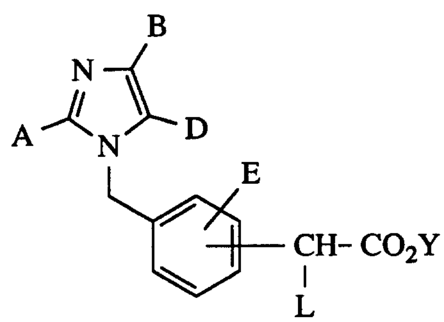

(IV),

in welcher

A, B, D, E, L und Z die oben angegebene Bedeutung haben,

umsetzt und gegebenenfalls nach vorgeschalteter Verseifung und/oder Aktivierung anschließend mit Aminen der allgemeinen Formel (V)

$HNR^1R^2$ (V),

in welcher

$R^1$ und $R^2$ die oben angegebene Bedeutung haben,

gegebenenfalls in Anwesenheit einer Base und/oder eines Hilfsstoffes, beispielsweise eines Dehydratisierungsmittels, in inerten Lösemitteln amidiert,

und gegebenenfalls die Substituenten A, B, D und E nach üblichen Methoden, beispielsweise durch Reduktion, Oxidation, Alkylierung oder Hydrolyse einführt oder in andere Gruppen überführt

und gegebenenfalls die Isomeren trennt, und im Fall der Herstellung der Salze mit einer entsprechenden

Base oder Säure umsetzt.

Das erfindungsgemäße Verfahren kann durch folgendes Formelschema beispielhaft erläutert werden:

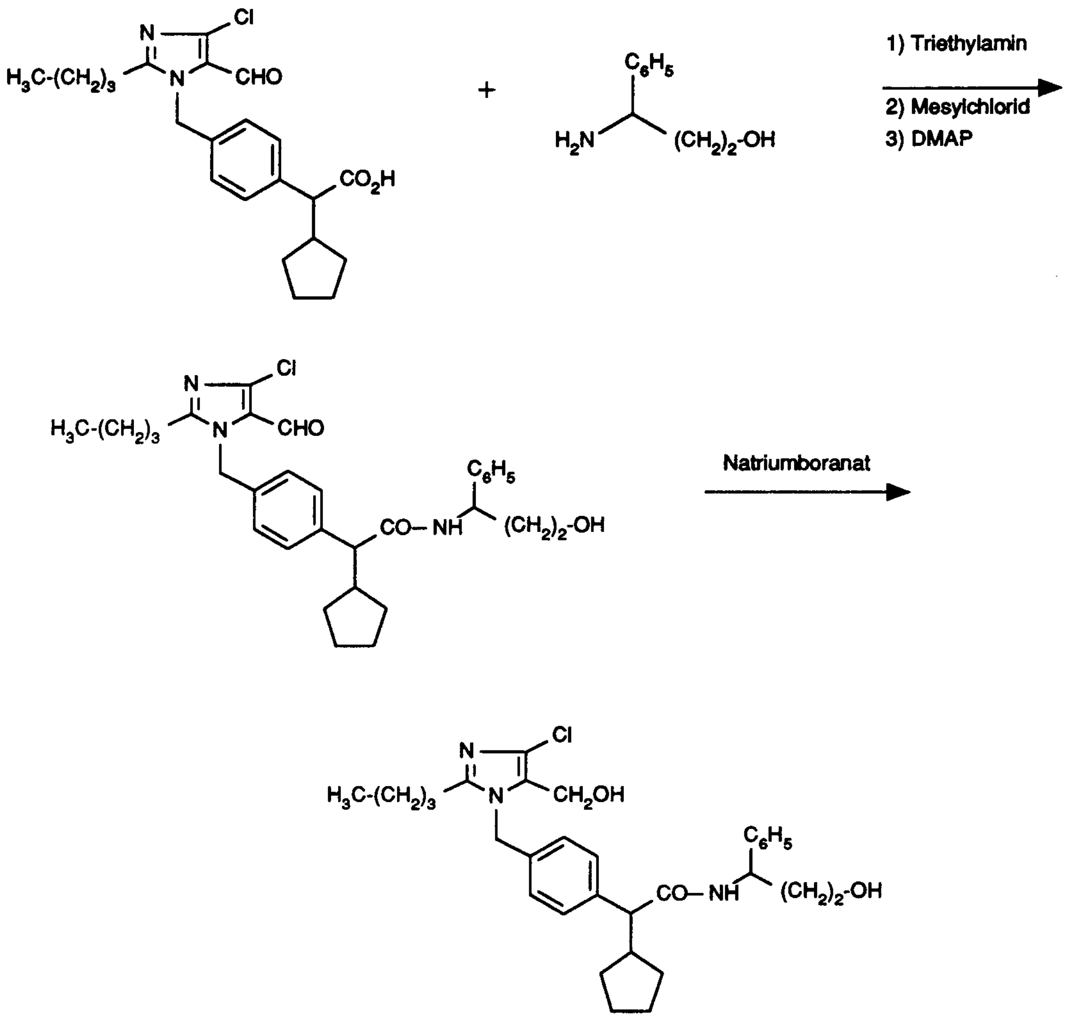


Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Dimethylformamid und Tetrahydrofuran.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl($C_1$-$C_6$)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle wie Natrium oder deren Hydride wie Natriumhydrid einzusetzen. Bevorzugt sind Natriumhydrid, Kaliumcarbonat, Triethylamin, Pyridin und Kalium-tert.butylat, DBU oder DABCO.

Im allgemeinen setzt man die Base in einer Menge von 0,05 mol bis 10 mol, bevorzugt von 1 mol bis 2 mol, bezogen auf 1 mol der Verbindung der Formel (III), ein.

Das erfindungsgemäße Verfahren wird im allgemeinen in einem Temperaturbereich von -30 °C bis +100 °C, bevorzugt von -10 °C bis +60 °C, durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Basen eignen sich für die Verseifung die üblichen anorganischen Basen. Hierzu gehören bevorzugt Alkalihydroxide oder Erdalkalihydroxide wie beispielsweise Lithiumhydroxid, Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Lithiumhydroxid, Natriumhydroxid oder Kaliumthydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung kann auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0 °C bis +100 °C, bevorzugt von +20 °C bis +80 °C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Bei der Durchführung der Reaktion entstehen im ersten Schritt die Carboxylate der erfindungsgemäßen Verbindungen als Zwischenprodukte, die isoliert werden können. Die erfindungsgemäßen Säuren erhält man durch Behandeln der Carboxylate mit üblichen anorganischen Säuren. Hierzu gehören bevorzugt Säuren wie beispielsweise Chlorwasserstoffsäure, Bromwasserstoffsäure, Schwefelsäure, Phosphorsäure oder Trifluoressigsäure. Es hat sich bei der Herstellung der Carbonsäuren hierbei als vorteilhaft erwiesen, die basische Reaktionsmischung der Verseifung in einem zweiten Schritt ohne Isolierung der Carboxylate anzusäuern.

Die Säuren können dann in üblicher Weise isoliert werden. Im Fall der basischen Heterocyclen können durch das Behandeln der Lösungen der Carboxylate mit den oben aufgeführten Säuren auch die Salze der Heterocyclen mit den anorganischen Säuren gewonnen werden.

Die Amidierung der Verbindungen der allgemeinen Formel (IV) erfolgt im allgemeinen in einem der oben aufgeführten Lösemitteln, vorzugsweise in Tetrahydrofuran oder Dichlormethan.

Die Amidierung kann gegebenenfalls über die aktivierte Stufe der Säurehalogenide [(IV) Y = Halogen], die aus den entsprechenden Säuren durch Umsetzung mit Thionylchlorid, Phosphortrichlorid, Phosphorpentachlorid, Phosphortribromid oder Oxalylchlorid hergestellt werden können, verlaufen.

Die Amidierung erfolgt im allgemeinen in einem Temperaturbereich von - 20 °C bis +80 °C, vorzugsweise von -10 °C bis +30 °C und Normaldruck.

Als Basen eignen sich dafür neben den oben aufgeführten Basen vorzugsweise Triethylamin und/oder Dimethylaminopyridin, DBU oder DABCO.

Die Base wird in einer Menge von 0,5 mol bis 10 mol, bevorzugt von 1 mol bis 5 mol, bezogen auf 1 mol der Verbindungen der allgemeinen Formeln (IV) und (V) eingesetzt.

Als säurebindende Mittel für die Amidierung können Alkali- oder Erdalkalicarbonate wie Natriumcarbonat, Kaliumcarbonat, Alkali- oder Erdalkalihydroxide wie beispielsweise Natrium- oder Kaliumhydroxid, oder organische Basen wie Pyridin, Triethylamin, N-Methylpiperidin, oder bicyclische Amidine wie 1,5-Diazabicyclo[3.4.0]-nonene-5 (DBN) oder 1,5-Diazabicyclo[3.4.0]undecene-5 (DBU) eingesetzt werden. Bevorzugt ist Kaliumcarbonat.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexylfluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid [vgl. J.C. Sheehan, S.L. LEdis, J. Am. Chem. Soc. 95, 875 (1973); F.E. Frerman et al., J. Biol. Chem. 225, 507 (1982) und N.B. Benoton, K.

Kluroda, Int. Pept. Prot. Res. 13, 403 (1979), 17, 187 (1981)].

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren eingesetzt.

Die oben aufgeführte Derivatisierung der Substituenten A, B, D und E erfolgt im allgemeinen nach literaturbekannten Methoden, wobei beispielhaft die Reduktion von Aldehyden oder Alkoxycarbonylverbindungen zu Alkoholen (a), die Reduktion von Doppelbindungen (b) und die Alkylierung (c) mit folgendem erläutert werden sollen:

a) Die Reduktion von Alkoxycarbonylverbindungen oder Aldehyden zu den entsprechenden Alkoholen erfolgt im allgemeinen mit Hydriden, wie Lithiumaluminiumhydrid oder Natriumborhydrid, bevorzugt mit Lithiumaluminumhydrid in inerten Lösemitteln wie Ethern, Kohlenwasserstoffen oder Alkoholen oder deren Gemischen, bevorzugt in Ethern wie beispielsweise Diethylether, Tetrahydrofuran oder Dioxan, oder Alkoholen wie Ethanol, im Fall der Aldehyde bevorzugt mit Natriumborhydrid in Ethanol, in einem Temperaturbereich von 0 °C bis +150 °C, bevorzugt von +20 °C bis +100 °C, bei Normaldruck.

Die Reduktion einer Doppelbindung erfolgt im allgemeinen durch Hydrierung mit Wasserstoff in Anwesenheit eines Katalysators wie beispielsweise Platin oder Platinoxide, Rhodium, Ruthenium, Chlorotris-(triphenylphosphin)rhodium, oder Palladium auf Tierkohle, bevorzugt mit Palladium auf Tierkohle in einem Temperaturbereich von 0 °C bis +150 °C, bevorzugt von +25 °C bis +100 °C.

b) Als Lösemittel für die Hydrierung eignen sich protische Lösemittel wie beispielsweise Methanol, Ethanol und/oder aprotische Lösemittel wie beispielsweise Tetrahydrofuran, Toluol, Dimethylformamid, Methylenchlorid, Dioxan oder Essigester.

Die Hydrierung wird bei einem Druck von 1 bis 300 atm, vorzugsweise bei 1 bis 20 atm durchgeführt.

c) Die Alkylierung erfolgt im allgemeinen in einem der oben aufgeführten Lösemitteln mit Alkylierungsmitteln wie beispielsweise ($C_1$-$C_8$)-Alkylhalogenide, Sulfonsäureester oder substituierte oder unsubstituierte ($C_1$-$C_6$)-Dialkyl- oder ($C_1$-$C_{10}$)-Diarylsulfate, vorzugsweise Methyljodid, p-Toluolsulfonsäureester oder Dimethylsulfat.

Die Verbindungen der allgemeinen Formel (II) sind teilweise bekannt und können hergestellt werden, indem man beispielsweise

Verbindungen der allgemeinen Formel (VI)

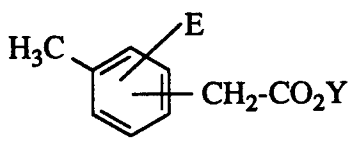 (VI),

in welcher

E und Y die oben angegebene Bedeutung haben,

zunächst mit Verbindungen der allgemeinen Formel (VII)

L-Z (VII),

in welcher

L die oben angegebene Bedeutung hat,

und

Z für Halogen, vorzugsweise für Brom steht,

in inerten Lösemitteln, gegebenenfalls in Anwesenheit einer Base alkyliert,

und in einem zweiten Schritt an der Methylgruppe eine Bromierung, gegebenenfalls in Anwesenheit eines Katalysators, nach üblicher Methode durchführt.

Die Alkylierung erfolgt im allgemeinen in einem der oben aufgeführten Lösemittel, vorzugsweise in Dimethylformamid in einem Temperaturbereich von 0 °C bis +70 °C, vorzugsweise von 0 °C bis +30 °C und Normaldruck.

Als Starter (Katalysator) für die Bromierung eignen sich beispielsweise Azobisisobutyronitril, Dibenzoylperoxid, vorzugsweise Azobisisobutyronitril, wobei der Starter in einer Menge von 0,01 mol bis 0,1 mol, bevorzugt von 0,01 mol bis 0,05 mol, bezogen auf 1 mol der Verbindung der allgemeinen Formel (VI)

eingesetzt wird.

Die Verbindungen der allgemeinen Formel (VI) sind an sich bekannt oder können nach bekannten Methoden hergestellt werden [vgl. J. Chem. Soc., Perkin Trans. 1, (9), 1706 - 1707; J. Chem. Soc., Chem. Commun., (2), 167 - 168].

Die Verbindungen der allgemeinen Formel (VII) sind an sich bekannt [vgl. Beilstein 5, 19/5, 24/5, 29] oder können nach üblicher Methode aus den entsprechenden Alkoholen oder Cycloalkenen hergestellt werden.

Ebenso sind die Verbindungen der allgemeinen Formel (III) an sich bekannt [vgl. z.B. Beilstein 25, 163; 23, 45; US 4 355 040] oder können nach üblicher Methode hergestellt werden.

Die Verbindungen der allgemeinen Formel (IV) sind als konkrete Stoffvertreter neu und können nach dem oben beschriebenen Verfahren hergestellt werden.

Die Amine der allgemeinen Formel (V) sind bekannt oder können nach bekannten Verfahren hergestellt werden [vgl. z.B. Beilstein 11/104, R.V. Vitzgert, Uspekhi, Khimii 32, 3 (1963); Russian Chem. Rev. 32, 1 (1969); Beilstein 4, 87].

Die erfindungsgemäßen Verbindungen der allgemeinen Formel (I) zeigen ein nicht vorhersehbares, wertvolles pharmakologisches Wirkspektrum.

Die erfindungsgemäßen Verbindungen besitzen eine spezifische A II-antagonistische Wirkung, da sie kompetitiv die Bindung von Angiotensin II an die Rezeptoren hemmen. Sie unterdrücken die vasokonstriktorischen und Aldosteronsekretionsstimulierenden Effekte des Angiotensin II. Darüberhinaus inhibieren sie die Proliferation von glatten Muskelzellen.

Sie können deshalb in Arzneimitteln zur Behandlung der arteriellen Hypertonie und Atherosklerose eingesetzt werden. Darüberhinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogen-begaster Krebs-Henseleit-Nährlösung folgender Zusammensetzung: 119 mmol/l NaCl; 2,5 mmol/l $CaCl_2$ x 2 $H_2O$; 1,2 mmol/l $KH_2PO_4$; 10 mmol/l Glucose; 4,8 mmol/l KCl; 1,4 mmol/l $MgSO_4$ x 7 $H_2O$ und 25 mmol/l $NaHCO_3$ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

Agonisten und ihre Standardkonzentrationen

Applikationsvolumen pro Einzelgabe = 100 µl):

| | | |
|---|---|---|
| KCl | 22,7;32,7;42,7;52,7 | mmol/l |
| 1Noradrenalin | $3x10^{-9}$;$3x10^{-8}$;$3x10^{-7}$;$3x10^{-6}$ | g/ml |
| Serotonin | $10^{-8}$;$10^{-7}$;$10^{-6}$;$10^{-5}$ | g/ml |
| B-HT 920 | $10^{-7}$;$10^{-6}$;$10^{-5}$ | g/ml |
| Methoxamin | $10^{-7}$;$10^{-6}$;$10^{-5}$ | g/ml |
| Angiotensin II | $3x10^{-9}$;$10^{-8}$;$3x10^{-8}$;$10^{-7}$ | g/ml |

Für die Berechnung der $IC_{50}$ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

## Tabelle A:

## Hemmung der Gefäßkontraktion an isolierten Aortenringen von Kaninchen in vitro

### $IC_{50}$ (nM) gegen Kontraktionen, induziert durch: AII

| Bsp.Nr.: | $IC_{50}$ [nM] |
|---|---|
| 11 | 930 |

Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht. Die Blutdruckveränderungen unter Substanzeinfluß sind als Mittelwerte ± SEM in der Tabelle angegeben.

Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht.

Der arterielle Blutdruck diese Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark und Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.
Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 $\mu$g), $^{3}$H-Angiotensin II (3-5 nM), Test-Pufferlösung (50 mM Tris, pH 7,2, 5 mM $MgCl_2$) sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu $K_i$- bzw. $IC_{50}$-Werten ($K_i$: für die verwendete Radioaktivität korrigierte $IC_{50}$-Werte; $IC_{50}$-Werte: Konzentration bei der die zu untersuchende Substanz eine 50%ige Hemmung der spezifischen Bindung des Radioliganden bewirkt).
Bsp.← 6: $K_i$ = 600 nM
Bsp.← 17: $K_i$ = 480 nM

Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium 199 mit 7,5% FCS und 7,5% NCS, 2 mM L-Glutamin und 15 mM HEPES, pH 7,4 in 5% $CO_2$ bei 37 °C kultiviert. Danach werden die Zellen durch Serumentzug für 2 - 3 Tage synchronisiert und sodann mit Serum oder anderen Faktoren zum Wachstum angeregt. Gleichzeitig werden Testverbindungen zugesetzt. Nach 16 - 20 Stunden wird 1 $\mu$Ci $^{3}$H-Thymidin zugefügt und nach weiteren 4 Stunden der Einbau dieser Substanz in die TCA-präzipitierbare DNA der Zellen bestimmt.

Zur Bestimmung der $IC_{50}$-Werte wird die Wirkstoffkonzentration errechnet, die bei sequentieller Verdünnung des Wirkstoffes halbmaximale Hemmung durch 10 % FCS hervorgerufener Thymidininkorporation bewirkt.
Bsp. 6 $IC_{50}$ = 100 nM
Bsp. 9 $IC_{50}$ = 38 nM
Bsp. 13 $IC_{50}$ = 28 nM.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Ausgangsverbindungen

Beispiel I

4-Methylphenylessigsäure-tert.butylester

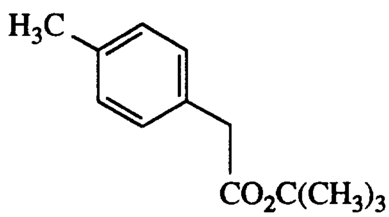

450 g (3 mol) 4-Methylphenylessigsäure, 1,13 l(12 mol) tert.Butanol und 90 g (0,74 mol) Dimethylaminopyridin werden in 2 l Dichlormethan gelöst. Nach Zugabe von 680 g (3,3 mol) Dicyclohexylcarbodiimid, gelöst in 400 ml Dichlormethan, wird 20 h bei 25 °C gerührt, der ausgefallene Harnstoff abgesaugt, mit 200 ml Dichlormethan gewaschen und die organische Phase je zweimal mit 500 ml 2 N Salzsäure und Wasser gewaschen. Die organische Phase wird eingeengt und destilliert.
Ausbeute: 408 g (66% der Theorie)
Siedepunkt: 73 - 78 °C/0,2 mm

Beispiel II

2-Cyclopentyl-2-(4-methylphenyl)essigsäure-tert.butylester

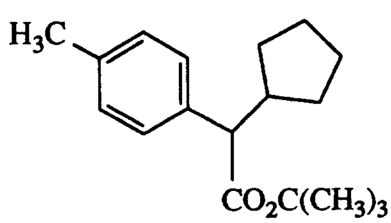

33,5 g (0,3 mol) Kalium-tert.butylat werden in 100 ml DMF unter Feuchtigkeitsausschluß bei 0 °C vorgelegt, und 51,6 g (0,25 mol) 4-Methylphenylessigsäure-tert.butylester in 250 ml DMF zugetropft. Es wird 30 min bei 0 °C gerührt und 32,2 ml (0,3 mol) Cyclopentylbromid in 150 ml DMF bei 5-15 °C zugetropft und 20 h bei 25 °C gerührt. Nach Einengen wird der Rückstand zwischen Wasser/Diethylether verteilt, die Etherphase über Natriumsulfat getrocknet und eingeengt. Das Produkt kristallisiert aus.
Ausbeute: 67g (97,5% der Theorie)
Festpunkt: 51 - 53 °C

Beispiel III

2-(4-Brommethyl-phenyl)-2-cyclopentyl-essigsäure-tert.butylester

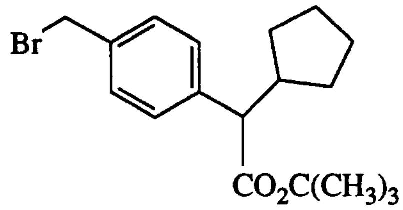

27,4 g (0,1 mol) 2-Cyclopentyl-2-(4-methylphenyl)-essigsäure-tert.butylester werden in 200 ml Tetrachlorkohlenstoff gelöst und zum Sieden erhitzt. Nach Zugabe von 0,82 g Azobisisobutyronitril werden 18,7 g (0,105 mol) N-Bromsuccinimid portionsweise zugegeben und anschließend 1 h refluxiert, auf 0 °C abgekühlt und vom Succinimid abfiltriert. Nach Einengen des Filtrates fällt das Produkt aus. Es wird mit Petrolether (40/60) gewaschen und getrocknet.
Ausbeute: 20 g (57% der Theorie)
Festpunkt: 73 - 76 °C

Beispiel IV

2-[4-(2-Butyl-4-chlor-5-formyl-imidazol-1-yl-methyl)phenyl]-2-cyclopentylessigsäure-tert.butylester

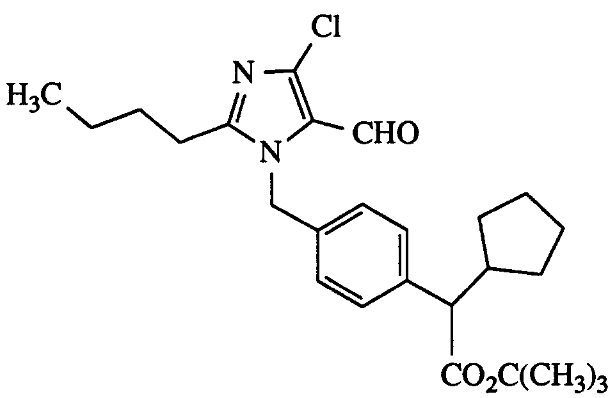

Unter Schutzgas werden 1,6 g (0,053 mol) Natriumhydrid (80%ig) in 50 ml DMF suspendiert, 10 g (0,053 mol) 2-Butyl-5-formyl-4-chlorimidazol (Herstellung nach EP 324 377) in 100 ml DMF bei 0 °C zugetropft, anschließend bei 0 °C 15 min gerührt und 18,9 g (0,053 mol) 2-(4-Brommethylphenyl)-2-cyclopentylessigsäure-tert.butylester in 100 ml DMF zugetropft. Es wird 2 h bei 0 °C nachgerührt, das Lösemittel abgedampft, der Rückstand in Diethylether aufgenommen, abfiltriert und nach Einengen über Kieselgel 60 mit Dichlormethan chromatographiert.
Ausbeute: 16,2 g (66,7% der Theorie)
Festpunkt: 101-102 °C

Beispiel V

2-[4-(2-Butyl-4-chlor-5-formyl-imidazol-1-yl-methyl)phenyl]-2-cyclopentyl-essigsäure

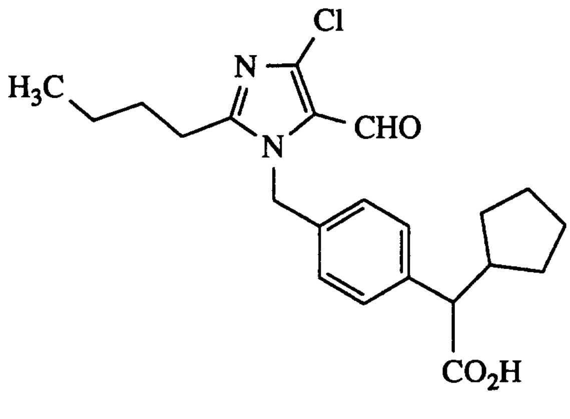

2,3 g (5 mmol) der Verbindung aus Beispiel IV werden in 5 ml Dichlormethan und 5 ml Trifluoressigsäure 5 h bei 25 ° C gerührt. Nach Einengen wird das Rohprodukt über Kieselgel 60 mit Dichlormethan/Methanol (100 : 5) chromatographiert.
Ausbeute: 1,8 g (87,6% der Theorie)
Festpunkt: 95-98 ° C

Herstellungsbeispiele

Beispiel 1

2-[4-(2-Butyl-4-chlor-5-formyl-imidazol-1-yl-methyl)-phenyl]-2-cyclopentyl-essigsäure-N-(3-hydroxy-1-phenylpropyl)amid

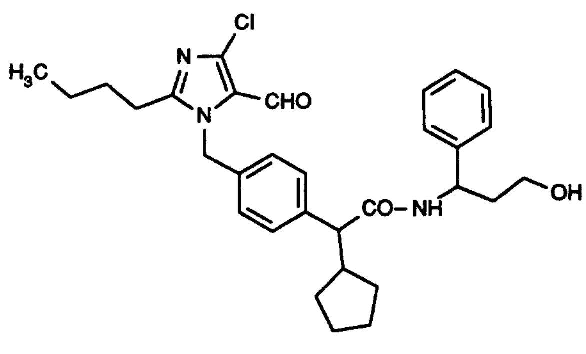

600 mg (1,49 mmol) der Verbindung aus Beispiel V werden in 5 ml THF gelöst und bei -30 ° C mit 0,41 ml (2,98 mmol) Triethylamin und 0,13 ml (1,64 mmol) Mesylchlorid versetzt und 30 min gerührt. Nach Zugabe von 0,18 mg (1,49 mmol) DMAP, 0,27 mg (1,79 mmol) 3-Amino-3-phenyl-propanol-1 in 5 ml THF wird 20 h bei 25 ° C gerührt. Nach Zugabe von 20 ml Wasser wird mit 0,2 ml Eisessig angesäuert, dreimal mit 20 ml Essigester extrahiert, die organische Phase über Natriumsulfat getrocknet, eingeengt und der Rückstand über Kieselgel 60 mit Essigester/Petrolether [(1:2) über (1:1) bis zu reinem Essigester] chromatographiert.
Ausbeute: 385mg (48% der Theorie)
$R_f = 0,66$ (Dichlormethan: Methanol = 10:1)

Beispiel 2

2-[4-(2-Butyl-4-chlor-5-hydroxymethyl-imidazol-1-yl-methyl)phenyl]-2-cyclopentyl-essigsäure-N-(3-hydroxy-1-phenyl-propyl)amid

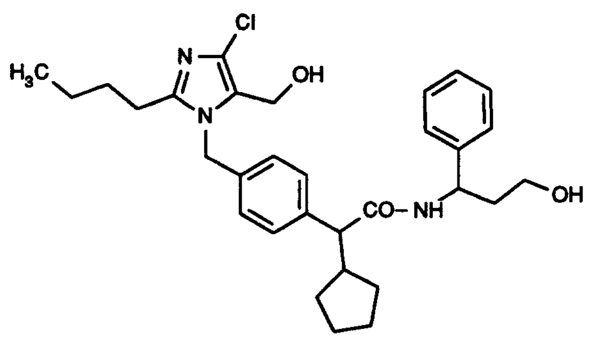

330 mg (0,61 mmol) der Verbindung aus Beispiel 1 werden in 20 ml Methanol gelöst und mit 24 mg (0,61 mmol) Natriumboranat umgesetzt. Nach 100 min werden 20 ml Wasser zugegeben, mit verdünnter Salzsäure angesäuert und zweimal mit 20 ml Essigester extrahiert. Die organische Phase wird über Natriumsulfat getrocknet und eingeengt.
Ausbeute: 310 mg (94% der Theorie)
$R_f$ = 0,37 (Dichlormethan : Methanol = 10:1)
In Analogie zu den Vorschriften der Beispiele 1 und 2 werden die in Tabelle 1, 2, 3 und 4 aufgeführten Beispiele jeweils in Abhängigkeit der Bedeutung von D hergestellt:

Tabelle 1:

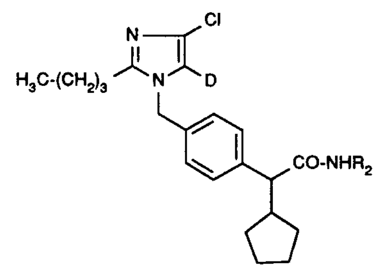

| Beispiel-Nr. | D | $R^2$ | $R_f$ / *Lösemittel | Isomer |
|---|---|---|---|---|
| 3 | -CHO | $-(CH_2)_3-OH$ | $0,50^G$ | rac |
| 4 | $-CH_2OH$ | $-(CH_2)_3-OH$ | $0,15^G$ | rac |
| 5 | -CHO | 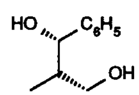 | $0,52^A$ | 2dia/ent |
| 6 | $-CH_2OH$ | 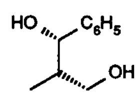 | $0,36 / 0,33^A$ | 2dia/ent |

Tabelle 2:

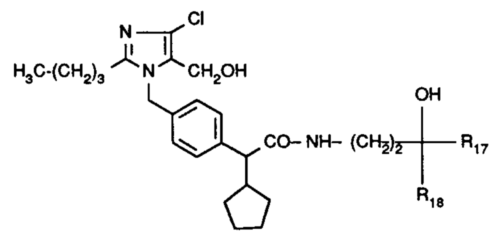

| Beispiel-Nr. | $R^{17}$ | $R^{18}$ | $R_f$ / * Lösemittel | Ausbeute (% d. Th.) | Isomer |
|---|---|---|---|---|---|
| 7 | H | $-C_6H_5$ | 0,42 C | 85,1 | 4dia |
| 8 | $-C_6H_5$ | $-C_6H_5$ | 0,46 C | 64,7 | rac |
| 9 | 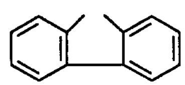 | | 0,44 C | 83,3 | rac |

Tabelle 3:

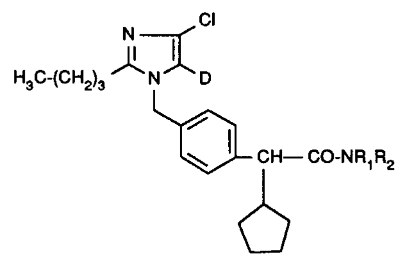

| Beispiel-Nr. | D | $R^1$ | $R^2$ | $R_f$/*Lösemittel | Isomer |
|---|---|---|---|---|---|
| 10 | 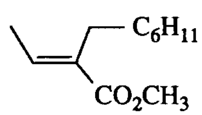 | H | 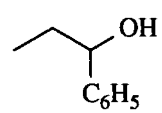 | $0,74^E$ | 4dia |
| 11 | 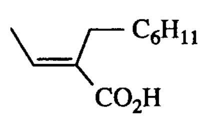 | H | 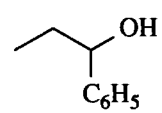 | $0,38^F$ | 4dia |

Tabelle 4:

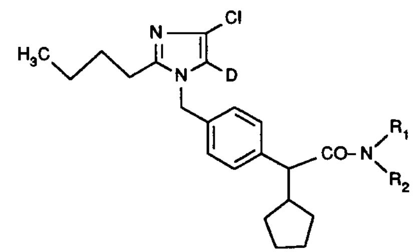

| Beispiel-Nr. | D | $R^1$ | $R^2$ | $R_f$/*Lösemittel | Isomer |
|---|---|---|---|---|---|
| 12 | CHO | H | 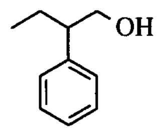 | 0,24 G | 4dia |
| 13 | $CH_2OH$ | H | 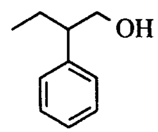 | 0,53 G | 4dia |
| 14 | CHO | H | 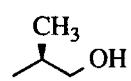 | 0,12 H | 2dia/ent |
| 15 | CHO | H | 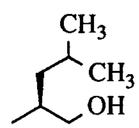 | 0,59 G | 2dia/ent |

Tabelle 4: (Fortsetzung)

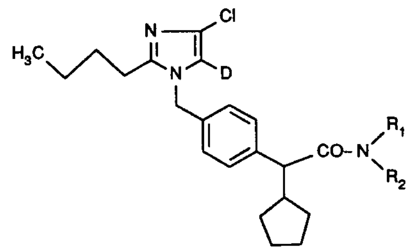

| Beispiel-Nr. | D | $R^1$ | $R^2$ | $R_f$/*Lösemittel | Isomer |
|---|---|---|---|---|---|
| 16 | $CH_2OH$ | H | 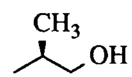 | 0,33 G | 2dia/ent |
| 17 | $CH_2OH$ | H | 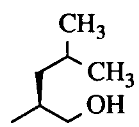 | 0,52 G | 2dia/ent |
| 18 | CHO | H | 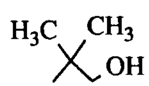 | 0,33 G | rac |
| 19 | $CH_2OH$ | H | 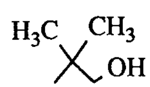 | 0,37 G | rac |

Lösemittelgemische

A = Dichlormethan : Methanol = 50:1

B = Petrolether : Essigester = 7:3

C = Toluol : Aceton = 1:1
D = Dichlormethan:Methanol = 9:1
E = Petrolether:Essigester = 3:7
F = Dichlormethan:Methanol:Eisessig = 9:1:0,1
G = Dichlormethan:Methanol = 10:1
H = Essigester:Petrolether = 1:1

Definition der Isomertypen:

4dia = Gemisch der vier möglichen Diastereomeren bei zwei Asymmetriezentren im Molekül
diaA/rac = racemisches Diastereomer mit dem größeren $R_f$-Wert
diaB/rac = racemisches Diastereomer mit dem kleineren $R_f$-Wert
diaA/ent = Diastereomer mit dem größeren $R_f$-Wert (ein Enantiomer)
diaB/ent = Diastereomer mit dem kleineren $R_f$-Wert (ein Enantiomer)
2dia/ent = Gemisch zweier enantiomerenreiner Diastereomeren
rac = Racemat
ent = Enantiomer

**Patentansprüche**

**1.** Imidazolyl-substituierte Phenylessigsäureamide der allgemeinen Formel

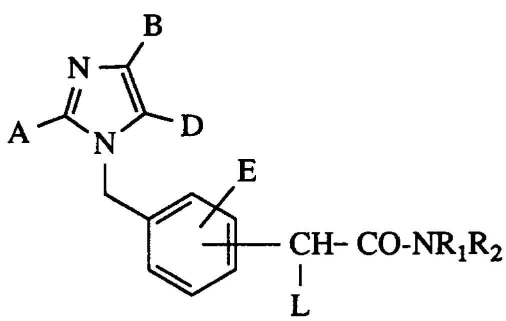

(I),

in welcher

A für geradkettiges oder verzweigtes Alkyl oder Alkenyl mit jeweils bis zu 8 Kohlenstoffatomen steht, oder für Cycloalkyl mit 3 bis 8 Kohlenstoffatomen steht,

B für Wasserstoff, Halogen oder für Perfluoralkyl mit bis zu 5 Kohlenstoffatomen steht,

D für eine Gruppe der Formel $-CH_2OR^3$, $-CO-R^4$, $-CO-NR^5R^6$,

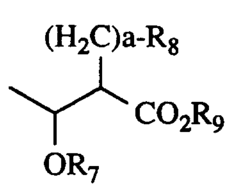

oder

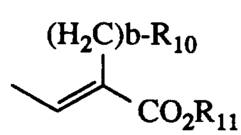

steht,
worin

$R^3$ Wasserstoff oder geradkettiges oder verzweigtes Alkyl mit bis zu 8 Kohlenstoffatomen bedeutet,

$R^4$ Wasserstoff, Hydroxy oder geradkettiges oder verzweigtes Alkoxy mit bis zu 8 Kohlenstoffatomen bedeutet,

$R^5$ und $R^6$ gleich oder verschieden sind und Wasserstoff, geradkettiges oder verzweigtes Alkyl mit bis zu 8 Kohlenstoffatomen bedeuten,

oder

$R^5$ die oben angegebene Bedeutung hat

und

$R^6$ eine Gruppe der Formel $-SO_2R^{12}$ bedeutet,

worin

$R^{12}$ geradkettiges oder verzweigtes Alkyl mit bis zu 6 Kohlenstoffatomen, Benzyl oder Phenyl bedeutet, die gegebenenfalls durch geradkettiges oder verzweigtes Alkyl mit bis zu 6 Kohlenstoffatomen substituiert sind,

a und b gleich oder verschieden sind und eine Zahl 0, 1 oder 2 bedeuten,

$R^7$ Wasserstoff oder geradkettiges oder verzweigtes Alkyl mit bis zu 8 Kohlenstoffatomen oder eine Hydroxyschutzgruppe bedeutet,

$R^8$ und $R^{10}$ gleich oder verschieden sind und Wasserstoff, geradkettiges oder verzweigtes Alkyl mit bis zu 6 Kohlenstoffatomen, Cycloalkyl mit 3 bis 8 Kohlenstoffatomen, Phenyl oder Thienyl bedeuten,

$R^9$ und $R^{11}$ gleich oder verschieden sind und Wasserstoff oder geradkettiges oder verzweigtes Alkyl mit bis zu 8 Kohlenstoffatomen bedeuten,

E für Wasserstoff, Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano oder Carboxy steht,

L für geradkettiges oder verzweigtes Alkyl mit bis zu 8 Kohlenstoffatomen steht, das gegebenenfalls durch Cycloalkyl mit 3 bis 8 Kohlenstoffatomen oder Phenyl substituiert ist,

für Cycloalkyl mit 3 bis 12 Kohlenstoffatomen steht, das gegebenenfalls durch geradkettiges oder verzweigtes Alkyl mit bis zu 6 Kohlenstoffatomen substituiert ist,

$R^1$ für Wasserstoff oder für geradkettiges oder verzweigtes Alkyl mit bis zu 8 Kohlenstoffatomen steht,

$R^2$ für einen Rest der Formel $-C(CH_3)_2-CH_2OH$,

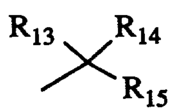 , 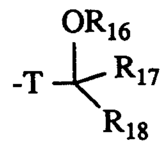 , 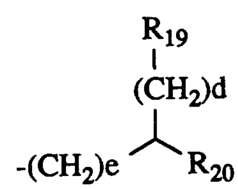 ,

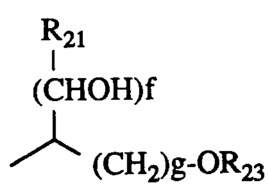 oder 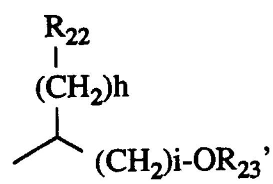

steht,

worin

$R^{13}$ und $R^{14}$ gleich oder verschieden sind und Wasserstoff oder geradkettiges oder verzweigtes Alkyl mit bis zu 8 Kohlenstoffatomen bedeuten,

$R^{15}$ geradkettiges oder verzweigtes Alkyl mit 2 bis 8 Kohlenstoffatomen bedeutet, das, gegebenenfalls auch bis zu 3-fach gleich oder verschieden durch Hydroxy, Carboxy, Trifluormethyl, Halogen, Nitro, Cyano, durch geradkettiges oder verzweigtes Alkoxy, Acyl oder Alkoxycarbonyl mit jeweils bis zu 8 Kohlenstoffatomen oder durch Aryl mit 6 bis 10 Kohlenstoffatomen oder einen 5- bis 7-gliedrigen, gesättigten oder ungesättigten Heterocyclus oder benzokondensierten Heterocyclus mit bis zu 3 Heteroatomen aus der Reihe S, N oder O substituiert sein muß, welche ihrerseits bis zu 2-fach gleich oder verschieden Halogen, Nitro, Cyano, Hydroxy oder durch geradkettiges oder verzweigtes

Alkyl oder Alkoxy mit jeweils bis zu 6 Kohlenstoffatomen substituiert sein können, oder
Alkyl gegebenenfalls durch eine Gruppe der Formel $-CO-NR^{24}R^{25}$ substituiert ist,
worin

| | |
|---|---|
| $R^{24}$ und $R^{25}$ | die oben angegebene Bedeutung von $R^5$ und $R^6$ haben und mit dieser gleich oder verschieden sind, oder |
| $R^{24}$ und $R^{25}$ | gemeinsam mit dem Stickstoffatom einen 5- bis 7-gliedrigen, gesättigten oder ungesättigten Heterocyclus mit bis zu 2 weiteren Heteroatomen aus der Reihe S, N oder O bilden, oder |
| $R^{15}$ | geradkettiges oder verzweigtes Alkoxy oder Alkoxycarbonyl mit jeweils bis zu 8 Kohlenstoffatomen, Hydroxy, Carboxy, Trifluormethyl oder die Gruppe der Formel $-CO-NR^{24}R^{25}$ bedeutet, worin |
| $R^{24}$ und $R^{25}$ | die oben angegebene Bedeutung haben, |
| T | geradkettiges oder verzweigtes Alkyl mit 2 bis zu 8 Kohlenstoffatomen bedeutet, |
| $R^{16}$ | Wasserstoff oder geradkettiges oder verzweigtes Alkyl mit bis zu 8 Kohlenstoffatomen bedeutet, |
| $R^{17}$ und $R^{18}$ | gleich oder verschieden sind und Wasserstoff, geradkettiges oder verzweigtes Alkyl mit bis zu 6 Kohlenstoffatomen oder Phenyl bedeuten, oder |
| $R^{17}$ und $R^{18}$ | gemeinsam einen gesättigten und/oder ungesättigten carbocyclischen Bi- oder Tricyclus bilden, |
| d | eine Zahl 0, 1, 2, 3 oder 4 bedeutet, |
| e | eine Zahl 1, 2, 3 oder 4 bedeutet, |
| f | eine Zahl 1, 2, 3 oder 4 bedeutet, |
| g | eine Zahl 1, 2, 3, 4, 5 oder 6 bedeutet, |
| h | eine Zahl 0, 1, 2, 3 oder 4 bedeutet, |
| i | eine Zahl 2, 3, 4, oder 5 bedeutet, |
| $R^{20}$ | die oben angegebene Bedeutung von $R^{15}$ hat und mit dieser gleich oder verschieden ist oder die $-CH_2OH$-Gruppe bedeutet, |
| $R^{19}$, $R^{21}$ und $R^{22}$ | gleich oder verschieden sind und Phenyl bedeuten, das gegebenenfalls bis zu 3-fach gleich oder verschieden durch Halogen, Hydroxy, Trifluormethyl oder durch geradkettiges oder verzweigtes Alkyl oder Alkoxy mit jeweils bis zu 8 Kohlenstoffatomen, Phenoxy oder Benzyloxy substituiert ist, |
| $R^{23}$ und $R^{23'}$ | gleich oder verschieden sind und Wasserstoff, geradkettiges oder verzweigtes Alkyl oder Alkoxycarbonyl mit jeweils bis zu 8 Kohlenstoffatomen bedeuten, |

und deren Salze.

**2.** Imidazolyl-substituierte Phenylessigsäurederivate nach Anspruch 1, wobei

| | |
|---|---|
| A | für geradkettiges oder verzweigtes Alkyl oder Alkenyl mit jeweils bis zu 6 Kohlenstoffatomen steht, oder für Cyclopropyl, Cyclobutyl, Cyclopentyl, Cyclohexyl oder Cycloheptyl steht, |
| B | für Wasserstoff, Fluor, Chlor, Brom oder für Perfluoralkyl mit bis zu 4 Kohlenstoffatomen steht, |
| D | für eine Gruppe der Formel $-CH_2OR^3$, $-CO-R^4$, $-CO-NR^5R^6$, |

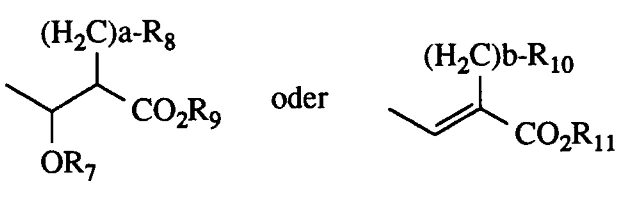

steht,
worin

$R^3$ Wasserstoff oder geradkettiges oder verzweigtes Alkyl mit bis zu 6 Kohlenstoffatomen bedeutet,

$R^4$ Wasserstoff, Hydroxy oder geradkettiges oder verzweigtes Alkoxy mit bis zu 6 Kohlenstoffatomen bedeutet,

$R^5$ und $R^6$ gleich oder verschieden sind und Wasserstoff, geradkettiges oder verzweigtes Alkyl mit bis zu 6 Kohlenstoffatomen bedeuten,

oder

$R^5$ die oben angegebene Bedeutung hat

und

$R^6$ eine Gruppe der Formel $-SO_2R^{12}$ bedeutet,

worin

$R^{12}$ geradkettiges oder verzweigtes Alkyl mit bis zu 4 Kohlenstoffatomen, Benzyl oder Phenyl bedeutet, die gegebenenfalls durch geradkettiges oder verzweigtes Alkyl mit bis zu 4 Kohlenstoffatomen substituiert sind,

a und b gleich oder verschieden sind und eine Zahl 0 oder 1 bedeuten,

$R^7$ Wasserstoff oder geradkettiges oder verzweigtes Alkyl mit bis zu 6 Kohlenstoffatomen oder Acetyl bedeutet,

$R^8$ und $R^{10}$ gleich oder verschieden sind und geradkettiges oder verzweigtes Alkyl mit bis zu 4 Kohlenstoffatomen, Cyclopropyl, Cyclobutyl, Cyclopentyl, Cyclohexyl oder Cycloheptyl, Phenyl oder Thienyl bedeuten,

$R^9$ und $R^{11}$ gleich oder verschieden sind und Wasserstoff oder geradkettiges oder verzweigtes Alkyl mit bis zu 6 Kohlenstoffatomen bedeuten,

E für Wasserstoff, Fluor, Chlor, Brom, Trifluormethyl, Carboxy oder geradkettiges oder verzweigtes Alkyl, Alkoxy oder Alkoxycarbonyl mit jeweils bis zu 4 Kohlenstoffatomen steht,

L für geradkettiges oder verzweigtes Alkyl mit bis zu 6 Kohlenstoffatomen steht, das gegebenenfalls durch Cyclopropyl, Cyclobutyl, Cyclopentyl, Cyclohexyl, Cycloheptyl, Cyclooctyl oder Phenyl substituiert ist,

für Cyclopropyl, Cyclobutyl, Cyclopentyl, Cyclohexyl, Cycloheptyl oder Cyclooctyl steht, die gegebenenfalls durch geradkettiges oder verzweigtes Alkyl mit bis zu 4 Kohlenstoffatomen substituiert sind,

$R^1$ für Wasserstoff oder für geradkettiges oder verzweigtes Alkyl mit bis zu 6 Kohlenstoffatomen steht,

$R^2$ für einen Rest der Formel $-C(CH_3)_2-CH_2OH$,

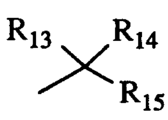
,
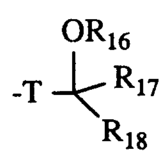
,
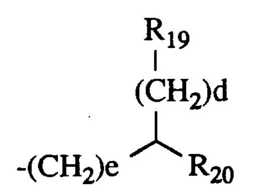
,

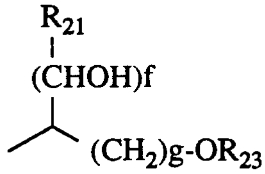
oder
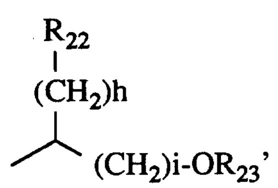

steht,

worin

$R^{13}$ und $R^{14}$ gleich oder verschieden sind und Wasserstoff oder geradkettiges oder verzweigtes Alkyl mit bis zu 6 Kohlenstoffatomen bedeuten,

$R^{15}$ geradkettiges oder verzweigtes Alkyl mit 2 bis 6 Kohlenstoffatomen bedeutet, das gegebenenfalls auch bis zu 2-fach gleich oder verschieden, durch Hydroxy, Carboxy, Trifluormethyl, Fluor, Chlor, Brom, durch geradkettiges oder verzweigtes Alkoxy, Acyl oder Alkoxycarbonyl mit jeweils bis zu 6 Kohlenstoffatomen oder durch Phenyl, Naphthyl, Furanyl, Pyrrolidinyl, Thienyl, Pyridyl, Tetrahydrofuranyl, Tetrahydropyranyl, Dihydrobenzopyranyl oder Dihydrobenzofuranyl substituiert sein muß, die gegebenenfalls durch Fluor, Chlor, Hydroxy substituiert sein können, oder
Alkyl gegebenenfalls durch eine Gruppe der Formel - $CO\text{-}NR^{24}R^{25}$ substituiert sein kann,
worin

| | |
|---|---|
| $R^{24}$ und $R^{25}$ | die oben angegebene Bedeutung von $R^5$ und $R^6$ haben und mit dieser gleich oder verschieden sind, oder |
| $R^{24}$ und $R^{25}$ | gemeinsam mit dem Stickstoffatom einen Morpholinring bilden, oder |
| $R^{15}$ | geradkettiges oder verzweigtes Alkoxy oder Alkoxycarbonyl mit jeweils bis zu 6 Kohlenstoffatomen, Hydroxy, Carboxy, Trifluormethyl oder die Gruppe der Formel $-CO\text{-}NR^{24}R^{25}$ bedeutet, worin |
| $R^{24}$ und $R^{25}$ | die oben angegebene Bedeutung haben, |
| T | geradkettiges oder verzweigtes Alkyl mit 2 bis zu 6 Kohlenstoffatomen bedeutet, |
| $R^{16}$ | Wasserstoff oder geradkettiges oder verzweigtes Alkyl mit bis zu 6 Kohlenstoffatomen bedeutet, |
| $R^{17}$ und $R^{18}$ | gleich oder verschieden sind Wasserstoff, geradkettiges oder verzweigtes Alkyl mit bis zu 4 Kohlenstoffatomen oder Phenyl bedeuten, oder |
| $R^{17}$ und $R^{18}$ | gemeinsam einen Indenyl- oder Fluorenylring bilden, |
| d | eine Zahl 0, 1, 2 oder 3 bedeutet, |
| e | eine Zahl 1, 2 oder 3 bedeutet, |
| f | eine Zahl 1, 2 oder 3 bedeutet, |
| g | eine Zahl 1, 2, 3, 4 oder 5 bedeutet, |
| h | eine Zahl 0, 1, 2 oder 3 bedeutet, |
| i | eine Zahl 2, 3 oder 4 bedeutet, |
| $R^{20}$ | die oben angegebene Bedeutung von $R^{15}$ hat und mit dieser gleich oder verschieden ist oder die $-CH_2OH$-Gruppe bedeutet, |
| $R^{19}$, $R^{21}$ und $R^{22}$ | gleich oder verschieden sind und Phenyl bedeuten, das gegebenenfalls bis zu 2-fach gleich oder verschieden durch Fluor, Chlor, Brom, Hydroxy, Trifluormethyl oder durch geradkettiges oder verzweigtes Alkyl oder Alkoxy mit jeweils bis zu 6 Kohlenstoffatomen, Phenoxy oder Benzyloxy substituiert ist, |
| $R^{23}$ und $R^{23'}$ | gleich oder verschieden sind und Wasserstoff oder geradkettiges oder verzweigtes Alkyl oder Alkoxycarbonyl mit jeweils bis zu 6 Kohlenstoffatomen bedeutet, |

und deren Salze.

**3.** Imidazolyl-substituierte Phenylessigsäureamide nach Anspruch 1
wobei

| | |
|---|---|
| A | für geradkettiges oder verzweigtes Alkyl oder Alkenyl mit jeweils bis zu 4 Kohlenstoffatomen steht, oder für Cyclopropyl, Cyclopentyl oder Cyclohexyl steht, |
| B | für Wasserstoff, Fluor, Chlor oder für Perfluoralkyl mit bis zu 2 Kohlenstoffatomen steht, |
| D | für eine Gruppe der Formel $-CH_2OR^3$, $-CO\text{-}R^4$, $-CO\text{-}NR^5R^6$, |

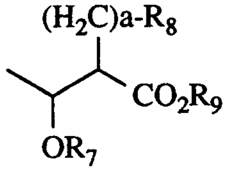

oder

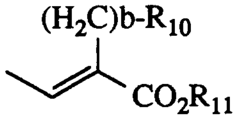

steht,
worin

$R^3$ Wasserstoff oder geradkettiges oder verzweigtes Alkyl mit bis zu 4 Kohlenstoffatomen bedeutet,

$R^4$ Wasserstoff, Hydroxy oder geradkettiges oder verzweigtes Alkoxy mit bis zu 4 Kohlenstoffatomen bedeutet,

$R^5$ und $R^6$ gleich oder verschieden sind und Wasserstoff, geradkettiges oder verzweigtes Alkyl mit bis zu 4 Kohlenstoffatomen bedeuten,

oder

$R^5$ die oben angegebene Bedeutung hat

und

$R^6$ eine Gruppe der Formel $-SO_2R^{12}$ bedeutet,

worin

$R^{12}$ Methyl, Ethyl, Benzyl, p-Tolyl oder Phenyl bedeutet,

a und b gleich oder verschieden sind und eine Zahl 0 oder 1 bedeuten,

$R^7$ Wasserstoff oder geradkettiges oder verzweigtes Alkyl mit bis zu 4 Kohlenstoffatomen bedeutet,

$R^8$ und $R^{10}$ gleich oder verschieden sind und Cyclopropyl, Cyclohexyl oder Phenyl bedeuten,

$R^9$ und $R^{11}$ gleich oder verschieden sind und Wasserstoff oder geradkettiges oder verzweigtes Alkyl mit bis zu 4 Kohlenstoffatomen bedeuten,

E für Wasserstoff, Fluor, Chlor, Brom, Trifluormethyl, Trifluormethoxy oder Methyl steht,

L für geradkettiges oder verzweigtes Alkyl mit bis zu 4 Kohlenstoffatomen steht, das gegebenenfalls durch Cyclopropyl, Cyclopentyl, Cyclohexyl, Cycloheptyl oder Phenyl substituiert ist,
für Cyclopropyl, Cyclopentyl, Cyclohexyl,Cycloheptyl oder Cyclooctyl steht,

$R^1$ für Wasserstoff oder für geradkettiges oder verzweigtes Alkyl mit bis zu 4 Kohlenstoffatomen steht,

$R^2$ für einen Rest der Formel $-C(CH_3)_2-CH_2OH$,

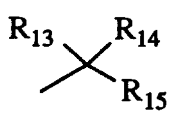 , 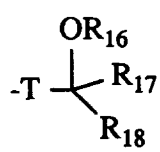 , 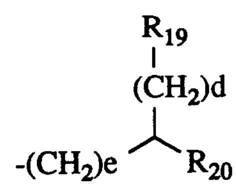 ,

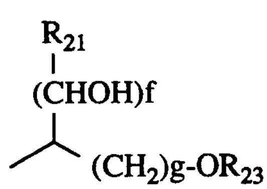 oder 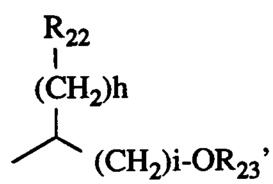

steht,
worin

$R^{13}$ und $R^{14}$ gleich oder verschieden sind und Wasserstoff oder geradkettiges oder verzweigtes Alkyl mit bis zu 4 Kohlenstoffatomen bedeuten,

$R^{15}$ geradkettiges oder verzweigtes Alkyl mit 2 bis 4 Kohlenstoffatomen bedeutet, das durch Hydroxy, Carboxy, Trifluormethyl, geradkettiges oder verzweigtes Alkoxy, Acyl oder Alkoxycarbonyl mit jeweils bis zu 4 Kohlenstoffatomen oder durch Phenyl, Pyridyl, Furanyl, Thienyl, Tetrahydrofuranyl oder Pyrrolidinyl substituiert sein muß, oder
geradkettiges oder verzweigtes Alkoxy oder Alkoxycarbonyl mit jeweils bis zu 4 Kohlenstoffatomen, Hydroxy, Carboxy oder Trifluormethyl bedeutet,

T geradkettiges oder verzweigtes Alkyl mit 2 bis zu 5 Kohlenstoffatomen bedeutet,

| | |
|---|---|
| $R^{16}$ | Wasserstoff oder geradkettiges oder verzweigtes Alkyl mit bis zu 4 Kohlenstoffatomen bedeutet, |
| $R^{17}$ und $R^{18}$ | gleich oder verschieden sind und Wasserstoff, Methyl, Ethyl oder Phenyl bedeuten, |
| | oder |
| $R^{17}$ und $R^{18}$ | gemeinsam einen Indenyl- oder Fluorenylring bilden, |
| d | eine Zahl 0, 1 oder 2 bedeutet, |
| e | eine Zahl 1, 2 oder 3 bedeutet, |
| f | eine Zahl 1, 2 oder 3 bedeutet, |
| g | eine Zahl 1, 2, 3 oder 4 bedeutet, |
| h | eine Zahl 0, 1 oder 2 bedeutet, |
| i | eine Zahl 2 oder 3 bedeutet, |
| $R^{20}$ | die oben angegebene Bedeutung von $R^{15}$ hat und mit dieser gleich oder verschieden ist oder die $-CH_2OH$-Gruppe bedeutet, |
| $R^{19}$, $R^{21}$ und $R^{22}$ | gleich oder verschieden sind und Phenyl bedeuten, das gegebenenfalls durch Fluor, Hydroxy, Trifluormethyl oder durch geradkettiges oder verzweigtes Alkyl oder Alkoxy mit jeweils bis zu 4 Kohlenstoffatomen substituiert ist, |
| $R^{23}$ und $R^{23'}$ | gleich oder verschieden sind und Wasserstoff oder geradkettiges oder verzweigtes Alkyl oder Alkoxycarbonyl mit jeweils bis zu 4 Kohlenstoffatomen bedeuten, |

und deren Salze.

**4.** Imidazolyl-substituierte Phenylessigsäurederivate nach Anspruch 1 zur therapeutischen Anwendung.

**5.** Verfahren zur Herstellung von Imidazolyl-substituierten Phenylessigsäurederivaten nach Anspruch 1, dadurch gekennzeichnet,
daß man
Verbindungen der allgemeinen Formel (II)

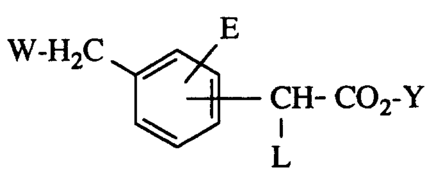

(II),

in welcher

| | |
|---|---|
| E und L | die in Anspruch 1 angegebene Bedeutung haben |
| W | für eine typische Abgangsgruppe wie beispielsweise Chlor, Brom, Jod, Tosylat oder Mesylat, vorzugsweise für Brom steht |

und

Y für $C_1$-$C_6$-Alkyl steht,

zunächst mit Imidazolen der allgemeinen Formel (III)

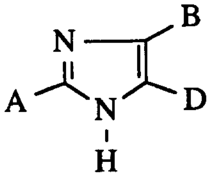

(III),

in welcher

A, B und D die in Anspruch 1 angegebene Bedeutung haben,

in inerten Lösemitteln, gegebenenfalls in Anwesenheit einer Base und gegebenenfalls unter Schutzgasatmosphäre zu Verbindungen der allgemeinen Formel (IV)

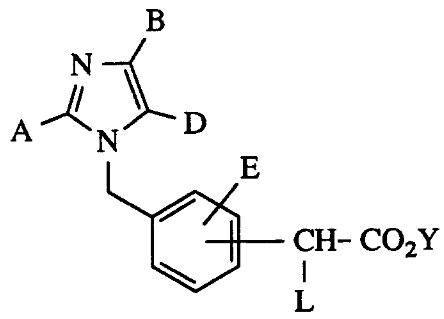

(IV),

in welcher

A, B, D, E, L und Z die in Anspruch 1 angegebene Bedeutung haben,

umsetzt und gegebenenfalls nach vorgeschalteter Verseifung und/oder Aktivierung anschließend mit Aminen der allgemeinen Formel (V)

$HNR^1R^2$ (V),

in welcher

$R^1$ und $R^2$ die in Anspruch 1 angegebene Bedeutung haben,

gegebenenfalls in Anwesenheit einer Base und/oder eines Hilfsstoffes, beispielsweise eines Dehydratisierungsmittels, in inerten Lösemitteln amidiert,

und gegebenenfalls die Substituenten A, B, D und E nach üblichen Methoden, beispielsweise durch Reduktion, Oxidation, Alkylierung oder Hydrolyse einführt oder in andere Gruppen überführt

und gegebenenfalls die Isomeren trennt, und im Fall der Herstellung der Salze mit einer entsprechenden Base oder Säure umsetzt.

**6.** Verfahren nach Anspruch 5, dadurch gekennzeichnet, daß man die Amidierung in einem Temperaturbereich von -20 °C bis +80 °C durchführt.

**7.** Arzneimittel enthaltend mindestens ein Imidazolyl-substituiertes Phenylessigsäurederivat nach Anspruch 1.

**8.** Arzneimittel nach Anspruch 7 zur Behandlung von Hypertonie und Atherosklerose.

**9.** Verfahren zur Herstellung von Arzneimitteln nach Anspruch 7, dadurch gekennzeichnet, daß man die Imidazolyl-substituierten Phenylsäureessigsäurederivate gegebenenfalls mit Hilfe von üblichen Hilfs- und Trägerstoffen in eine geeignete Applikationsform überführt.

**10.** Verwendung von Imidazolyl-substituierten Phenylessigsäurederivaten zur Herstellung von Arzneimitteln.

Europäisches Patentamt

# EUROPÄISCHER RECHERCHENBERICHT

Nummer der Anmeldung

EP 93 10 3217

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
| P,X | EP-A-0 513 533 (BAYER AG)<br>19. November 1992<br>* Beispiele V,XLVI,LXIV,LXV,XCVI,XCVIII, CLX-CLXVI,CLXIX-CLXXIV,CLXXVII-CLXXXII, CLXXVI,CLXXXIII-CLXXXVII,CCVII-CCXXXI,CCXL ,CCXLIII-CCXLVIII,CCLIII-CCLVIII *<br>* Ansprüche 1-9 * | 1-5,7-10 | C07D233/68<br>A61K31/415<br>// C07D233/66<br>C07D233/92 |
| P,Y | * das ganze Dokument *<br>--- | 1-4,7-10 | |
| Y | WO-A-9 112 002 (MERCK & CO.,INC.)<br>22. August 1991<br>* Ansprüche 1,7,9 * | 1-4,7-10 | |
| X | * Anspruch 1 und Ansprüche 7-12 *<br>--- | 10 | |
| Y | JOURNAL OF MEDICINAL CHEMISTRY<br>Bd. 33, Nr. 5, Mai 1990,<br>Seiten 1312 - 1329<br>DUNCIA J.V. ET AL. 'The discovery of potent nonpeptide angiotensin II receptor antagonists: A new class of potent antihypertensives'<br>* das ganze Dokument * | 1-4,7-10 | RECHERCHIERTE SACHGEBIETE (Int. Cl.5 ) |
| X | * Seite 1316; Beispiel 10 *<br>--- | 10 | C07D<br>A61K |
| A | EP-A-0 324 377 (E.I. DU PONT DE NEMOURS AND COMPANY)<br>19. Juli 1989<br>* das ganze Dokument *<br>----- | 1-10 | |

Der vorliegende Recherchenbericht wurde für alle Patentansprüche erstellt

| Recherchenort | Abschlußdatum der Recherche | Prüfer |
|---|---|---|
| MUENCHEN | 04 JUNI 1993 | HARTRAMPF G.W. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)